# FASCICULE DE BREVET EUROPEEN

(11) **EP 3 009 153 B1**
(45) Date de publication et mention de la délivrance du brevet: **04.08.2021**
(21) Numéro de dépôt: 15190188.1
(22) Date de dépôt: 16.10.2015
(51) Int. Cl.: A61L 27/06

(54) **RESSORT D'ORTHODONTIE HÉLICOÏDAL ET SON PROCÉDÉ DE FABRICATION**
KIEFERORTHOPÄDIE SCHRAUBENFEDER UND VERFAHREN ZUR IHRE HERSTELLUNG
ORTHODONTICS HELICAL SPRING AND METHOD OF PRODUCTION

(30) Priorité: 17.10.2014 FR 1459976
(43) Date de publication de la demande: 20.04.2016
(73) Titulaire: Université de Lorraine, 54052 Nancy Cedex (FR); L'école Supérieure Nationale d'Arts et Métiers (ENSAM), 75013 Paris (FR)
(72) Inventeur: FILLEUL, Marie-Pierryle, 95300 Pontoise (FR); LAHEURTE, Pascal, 57600 Forbach (FR); TERRASSE, Jean-Maxime, 57000 Metz (FR); GEORGE, Olivier, 54140 Jarville La Malgrange (FR); PELTIER, Laurent, 57645 Nouilly (FR)
(74) Mandataire: Novagraaf Technologies

(56) Documents cités:
- WO-A1-2013/068366
- WO-A1-2013/068691
- JP-A- 2008 220 811
- HAO ET AL: "Elastic deformation behaviour of Ti-24Nb-4Zr-7.9Sn for biomedical applications", ACTA BIOMATERIALIA, ELSEVIER, AMSTERDAM, NL, vol. 3, no. 2, 13 février 2007 (2007-02-13), pages 277-286, XP005886205, ISSN: 1742-7061, DOI: 10.1016/J.ACTBIO.2006.11.002
- SUN F ET AL: "Influence of a short thermal treatment on the superelastic properties of a titanium-based alloy", SCRIPTA MATERIALIA, ELSEVIER, AMSTERDAM, NL, vol. 63, no. 11, 1 novembre 2010 (2010-11-01), pages 1053-1056, XP027289858, ISSN: 1359-6462 [extrait le 2010-08-02]

## Description

### Domaine technique

La présente invention se rapporte à un ressort d'orthodontie hélicoïdal. Elle concerne également un procédé de fabrication d'un tel ressort.

### Etat de la technique

Dans le domaine de l'orthodontie, il est de pratique courante de placer sur les dents d'un patient des accessoires permettant d'exercer des tensions permanentes sur de longues périodes (quelques semaines à quelques mois) afin de provoquer un déplacement d'une ou plusieurs dents dans la mâchoire. La dent se déplace dans l'os alvéolaire, accompagnée de son ligament. Afin de permettre ces mouvements, un groupe de cellules ostéoblastiques et ostéoclastiques est nécessaire pour effectuer le remodelage osseux autour de la dent. Le recrutement de ces cellules nécessaires au mouvement est permis par l'application de forces douces et régulières. A l'inverse, des forces d'intensité trop élevée et non régulières auront tendance à créer une phase de hyalinisation qui va stopper le déplacement et augmenter le risque d'apparition de résorptions radiculaires.

Les produits présents sur le marché sont :
- les chaînettes élastomériques
- les ressorts en acier
- les ressorts en nickel titane.

Il est montré que les chainettes élastomériques développent des forces qui vont diminuer rapidement après leur mise en place. Leur temps d'action efficace ne va pas dépasser 48 heures. Les forces qui vont être appliquées sont donc discontinues. Il est donc nécessaire de remplacer rapidement et régulièrement ces chaînettes.

Il a été montré par ailleurs que les ressorts en acier développent des forces trop importantes, leur utilisation a donc été abandonnée.

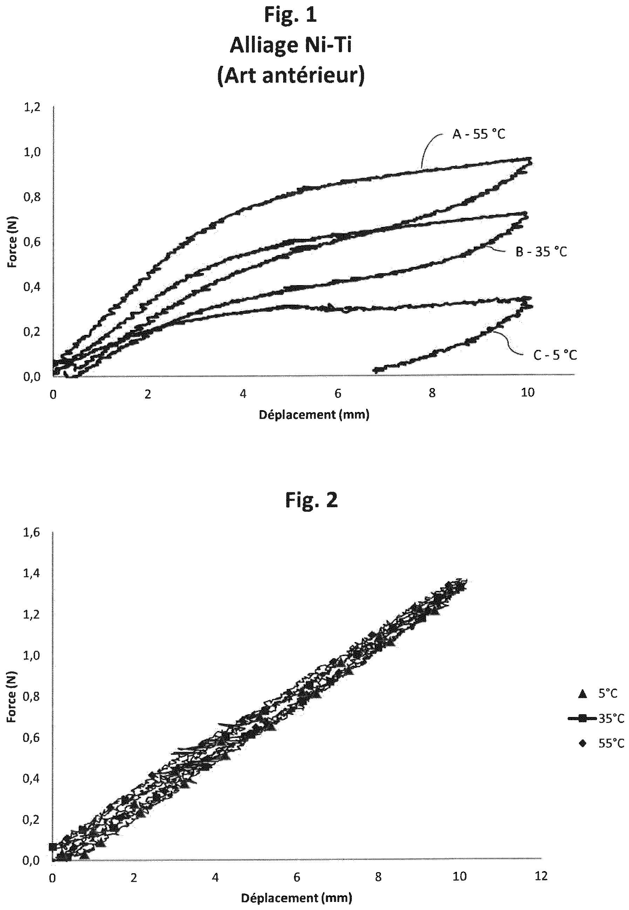

Les ressorts en nickel titane développent des forces dites douces dans la mesure où ils sont aptes à développer une force qui varie peu en fonction de l'allongement du ressort. Ceci lui permet d'agir sur de longues périodes sans qu'il soit nécessaire d'intervenir et modifier l'appareillage. Cependant, on constate que la température intrabuccale a une influence importante sur le comportement du ressort. Il est prouvé que la température intrabuccale est très variable. Des études ont montrées qu'elle variait entre 5°C et 55°C au cours des habitudes alimentaires des patients en fonction des aliments mis en bouche. Par exemple une boisson chaude fait monter fortement la température. Au contraire, la consommation d'une crème glacée la fait descendre de manière importante. Or, les ressorts en nickel titane sont sensibles aux variations de température intrabuccales. Des essais de traction sur un ressort en alliage de nickel-titane tel que commercialisé actuellement, ont été effectués à différents niveaux de température, à savoir à 5°C, à 35°C et à 55°C. Chaque essai correspond à une phase de traction jusqu'à 10 mm et à un relâchement. Les résultats sont montrés sur la figure 1. Les courbes A, B et C correspondent à une température d'essai respectivement à 55°C, 35°C et 5°C. On remarque que les efforts de traction développés par le ressort peuvent varier de 0,2 à 0,9 N pour un même allongement de 8 mm, ce qui est une variation considérable. La non régularité des tensions ainsi induites perturbent le processus de reconstruction qui permet le déplacement de la dent.

De plus, certaines personnes peuvent développer des allergies au nickel, ce qui leur interdit l'utilisation d'une telle solution technique.

Pour améliorer une biocompatibilité chimique des ressorts dentaires l'une des solutions consiste à les fabriquer à partir des alliages à base de titane décrits, par exemple, dans les demandes de brevet internationales WO2013/068366A1 et WO2013/068691 A1, qui ne comportent que des éléments biocompatibles chimiquement.

L'invention vise à fournir un ressort d'orthodontie hélicoïdal développant des forces invariantes avec la température intrabuccale. Elle vise également à fournir un procédé de fabrication d'un tel ressort.

### Description de l'invention

Avec ces objectifs en vue, l'invention a pour objet un ressort hélicoïdal d'orthodontie, caractérisé en ce qu'il est réalisé en alliage de titane β métastable, l'alliage comportant du niobium, du zirconium, et de l'oxygène, caractérisé en ce que l'alliage de titane comporte du niobium dans un rapport massique compris entre 25 et 45%, du zirconium dans un rapport massique compris entre 0 et 20% et de l'oxygène dans un rapport massique inférieur à 1,5%, la structure cristallographique de l'alliage étant une phase martensitique avec une présence de phase austénitique et des précipités de phase ω.

Les inventeurs ont constaté, que les ressorts en alliage de titane β métastable au niobium ou au molybdène présentaient une stabilité de la force dans la plage de température intéressante en orthodontie, à savoir sur la plage de 5 à 55 °C. Les variations constatées sur les forces appliquées lorsque la température varie sont inférieures à 10%, voire à quelques pourcents. Les inclusions de phases ω dans la structure cristalline apportent une grande résistance mécanique à l'alliage, tout en conservant ses propriétés de faible module d'élasticité.

Selon un mode de réalisation particulier, le ressort comporte des attaches rapportées par vissage à chaque extrémité. On peut ainsi mettre le ressort en traction sans réaliser de forme particulière aux extrémités du ressort mais en rapportant mécaniquement les attaches en prise avec quelques spires du ressort. Le ressort peut être fabriqué en grande longueur et coupé à la longueur souhaitée au moment de la mise en œuvre par le praticien.

L'invention a aussi pour objet un procédé de fabrication d'un ressort hélicoïdal d'orthodontie, selon l'une quelconque des revendications 1 à 7, caractérisé en ce qu'il comprend:
- écrouissage à un taux supérieur à 20% d'un fil orthodontique en alliage de titane β métastable, l'alliage étant en forme d'un ingot comportant du niobium dans un rapport massique compris entre 25 et 45%, du zirconium dans un rapport massique compris entre 0 et 20% et de l'oxygène dans un rapport massique inférieur à 1,5%, la structure étant initialement une phase austenitique ou une phase martensitique ou un mélange des deux phases,
- enroulement du fil orthodontique sur un mandrin à température ambiante,
- un traitement thermique comprenant un maintien en température du ressort sur le mandrin, la température de maintien étant comprise entre 250 et 550°C, pendant une durée inférieure à 10 minutes, suivi par une trempe du ressort, de manière à ce que l'alliage comprenne en outre après la trempe une phase ω en précipités,

L'écrouissage initial permet de favoriser la formation de phase martensitique dans la structure cristallographique de l'alliage. Comme cet alliage a des propriétés superélastiques, l'enroulement sur un mandrin n'est pas suffisant pour donner la forme du ressort. La forme hélicoïdale est acquise par l'alliage par le traitement thermique qui suit l'enroulement. Le maintien en température est de courte durée, ce qui permet d'amorcer la formation de phase ω. La trempe qui termine le traitement thermique arrête le développement de la phase oméga qui se trouve sous forme de précipités.

Selon un mode de réalisation, l'écrouissage est réalisé par une étape de tréfilage, précédée optionnellement par une étape de laminage.

Le traitement thermique de faible durée a pour effet d'augmenter la résistance de l'alliage, par la croissance de précipités de phase ω.

A l'étape d'écrouissage, on réalise par exemple une déformation vraie comprise entre 4 et 5. La déformation vraie est égale au logarithme naturel du quotient de la dimension après déformation et de la dimension initiale.

### Brève description des figures

L'invention sera mieux comprise et d'autres particularités et avantages apparaîtront à la lecture de la description qui va suivre, la description faisant référence aux dessins annexés parmi lesquels :
- la figure 1 est un diagramme montrant le comportant d'un alliage selon l'art antérieur ;
- la figure 2 est un diagramme similaire à la figure 1 d'un alliage selon un mode de réalisation de l'invention ;
- la figure 3 montre un ressort selon un mode de réalisation de l'invention
- la figure 4 montre un diagramme représentant le module d'élasticité et la résistance maximale d'un alliage selon un deuxième exemple de l'invention.

### DESCRIPTION DETAILLEE

Un ressort conforme à un mode de réalisation de l'invention comporte un fil 1 enroulé en forme hélicoïdale. Il peut être muni à chaque extrémité d'attaches 2. Chaque attache 2 comporte un logement 21 dans lequel l'extrémité du ressort est accrochée, et un œilleton 22 apte à être accroché à un crochet. Ainsi, le ressort peut être tendu en traction entre deux crochets disposés sur les dents d'un patient, d'une manière connue en soi.

Le fil 1 est fabriqué à partir d'un alliage comprenant en rapports massiques entre 25 et 45% de niobium, entre 0 et 20% de zirconium et moins de 1,5% d'oxygène. Il est coulé en lingot d'environ 20 x 20 x 70 MM. Ce lingot est homogénéisé pendant 10 h à 100 °C suivi d'une trempe à l'eau. Il est ensuite laminé jusqu'à une dimension d'environ 2 x 2 mm en plusieurs passes, puis tréfilé en plusieurs passes jusqu'à la dimension du fil orthodontique, soit un diamètre proche de 0,18 mm. Le fil ainsi hyperdéformé est ensuite enroulé sur un mandrin aux dimensions du ressort. Un traitement thermique de courte durée par effet Joule suit l'opération d'enroulement. Le traitement thermique amène le ressort à une température comprise entre 200 et 500 °C pendant une durée de 10 min.

Des essais de traction ont été conduits sur cet échantillon, avec une phase de traction suivie d'une phase de relâchement, à respectivement 5°C, 35°C et 55°C. Les résultats sont montrés sur la figure 2. Ils montrent que l'évolution de la force de traction en fonction de la température est à peine perceptible.

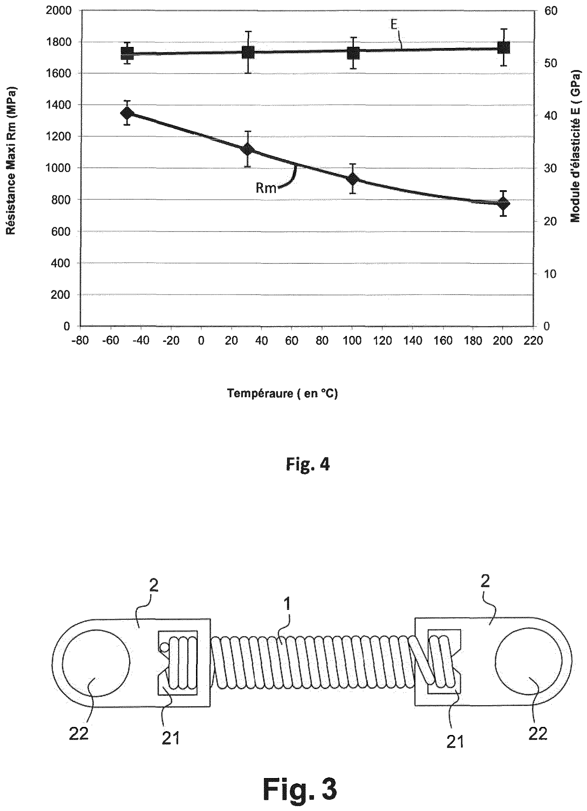

Selon un deuxième exemple, un alliage est élaboré de manière similaire, avec une composition de titane avec 26% de niobium, exprimé en pourcentage atomique. Des essais de caractérisation sont montrés sur la figure 4. Le diagramme de cette figure représente les résultats de caractérisation du module d'élasticité d'une part, et de la résistance maximale d'autre part, à différentes températures comprises entre -40 et 200°C. Les résultats montrent l'invariance du module d'élasticité en fonction de la température, y compris dans la zone de températures intrabuccales. Cet alliage est donc parfaitement adapté à la fabrication de ressorts hélicoïdaux d'orthodontie.

## Revendications

1. Ressort d'orthodontie réalisé en alliage de titane β métastable, l'alliage comportant du niobium, du zirconium et de l'oxygène, et optionnellement du molybdène et de l'étain, **caractérisé en ce que** le ressort d'orthodontie est hélicoïdal, et **en ce que** l'alliage de titane comporte du niobium dans un rapport massique compris entre 25 et 45%, du zirconium dans un rapport massique compris entre 0 et 20% et de l'oxygène dans un rapport massique inférieur à 1,5%, la structure cristallographique de l'alliage étant une phase martensitique avec une présence de phase austénitique et des précipités de phase ω.

2. Ressort d'orthodontie selon la revendication 1, **caractérisé en ce qu'**il est adapté à fonctionner avec des variations de forces de traction inférieures à 10% dans une plage de température de 5°C à 55°C.

3. Ressort d'orthodontie selon l'une quelconque des revendications 1 à 2, **caractérisé en ce qu'**il comporte deux extrémités opposées l'une de l'autre et des attaches (2) accrochées à chaque extrémité.

4. Ressort d'orthodontie selon la revendication 3, **caractérisé en ce que** chaque attache (2) est rapportée par vissage à l'extrémité correspondante du ressort d'orthodontie.

5. Ressort d'orthodontie selon la revendication 3, **caractérisé en ce que** chaque attache (2) comporte :
• un logement (21) dans lequel l'extrémité correspondante du ressort d'orthodontie est accrochée et
• un œilleton (22), apte à être accroché à un crochet.

6. Ressort d'orthodontie selon l'une quelconque des revendications 1 à 5, **caractérisé en ce qu'**il est constitué en un fil orthodontique (1) en alliage de titane β métastable, le fil orthodontique (1) étant enroulé en forme hélicoïdale.

7. Ressort d'orthodontie selon la revendication 6, **caractérisé en ce que** le fil orthodontique (1) a un diamètre de 0,18 mm.

8. Procédé de fabrication d'un ressort tel que défini selon l'une quelconque des revendications 1 à 7, **caractérisé en ce qu'**il comprend :
• une étape d'écrouissage à un taux supérieur à 20% d'un accessoire en alliage de titane β métastable, l'alliage étant en forme d'un lingot comportant du niobium dans un rapport massique compris entre 25 et 45%, du zirconium dans un rapport massique compris entre 0 et 20% et de l'oxygène dans un rapport massique inférieur à 1,5%, la structure étant initialement une phase austénitique ou une phase martensitique ou un mélange des deux phases,
• une étape de traitement thermique comprenant un maintien de l'accessoire en température comprise entre 250 et 550°C pendant une durée inférieure à 10 minutes, suivie par une trempe de l'échantillon de l'accessoire, de manière à ce que l'alliage comprenne en outre après la trempe une phase ω en précipités.
l'accessoire en alliage de titane β métastable étant constitué en un fil orthodontique (1), et
**en ce que** ledit procédé de fabrication comporte en outre une étape d'enroulement du fil orthodontique (1) sur un mandrin à température ambiante, et l'étape d'enroulement précédant l'étape de traitement thermique opérée lorsque le fil orthodontique se trouve sur le mandrin.

9. Procédé de fabrication selon la revendication 8, **caractérisé en ce que** l'étape d'écrouissage comporte une étape de tréfilage, précédée optionnellement par une étape de laminage.

10. Procédé de fabrication selon la revendication 8 ou 9, **caractérisé en ce qu'**il comporte en outre une étape d'homogénéisation du lingot pendant 10h à 100°C suivie d'une étape de trempe à l'eau du lingot homogénéisé.

11. Procédé de fabrication selon l'une quelconque des revendications 8 à 10, **caractérisé en ce que** l'étape d'écrouissage comporte une étape de déformation vraie comprise entre 4 et 5.

12. Procédé de fabrication selon l'une quelconque des revendications 8 à 11, **caractérisé en ce que** le ressort orthodontique hélicoïdal comporte deux extrémités opposées l'une de l'autre, et **en ce que** le procédé de fabrication comporte en outre une étape de vissage des attaches (2) à chaque extrémité du ressort orthodontique hélicoïdal.

## Patentansprüche

1. Kieferorthopädische Feder, die aus einer metastabilen β-Titanlegierung gefertigt ist, wobei die Legierung Niob, Zirkonium und Sauerstoff und optional Molybdän und Zinn aufweist, **dadurch gekennzeichnet, dass** die kieferorthopädische Feder schraubenförmig ist und dadurch, dass die Titanlegierung Niob in einem Massenverhältnis von 25 bis 45 %, Zirkonium in einem Massenverhältnis von 0 bis 20 %, und Sauerstoff in einem Massenverhältnis aufweist, das kleiner als 1,5 % ist, wobei die kristallografische Struktur der Legierung eine martensitische Phase mit einem Auftreten einer austenitischen Phase und Niederschlägen einer ω-Phase ist.

2. Kieferorthopädische Feder nach Anspruch 1, **dadurch gekennzeichnet, dass** sie geeignet ist, in einem Temperaturbereich von 5 °C bis 55 °C mit Zugkraftschwankungen, die kleiner als 10 % sind, arbeiten kann.

3. Kieferorthopädische Feder nach einem der Ansprüche 1 bis 2, **dadurch gekennzeichnet, dass** sie zwei einander gegenüberliegende Enden und an jedem Ende eingehakte Befestigungen (2) aufweist.

4. Kieferorthopädische Feder nach Anspruch 3, **dadurch gekennzeichnet, dass** jede Befestigung (2) durch eine Verschraubung an dem entsprechenden Ende der kieferorthopädischen Feder aufgeschraubt ist.

5. Kieferorthopädische Feder nach Anspruch 3, **dadurch gekennzeichnet, dass** jede Befestigung (2) Folgendes aufweist:
• ein Gehäuse (21), in dem das entsprechende Ende der kieferorthopädischen Feder eingehakt ist und
• eine Öse (22), die fähig ist, an einem Haken eingehakt zu werden.

6. Kieferorthopädische Feder nach einem der Ansprüche 1 bis 5, **dadurch gekennzeichnet, dass** sie aus einem kieferorthopädischen Draht (1) aus metastabiler β-Titanlegierung aufgebaut ist, wobei der kieferorthopädische Draht (1) in einer schraubenförmigen Form gewickelt ist.

7. Kieferorthopädische Feder nach Anspruch 6, **dadurch gekennzeichnet, dass** der kieferorthopädische Draht (1) einen Durchmesser von 0,18 mm besitzt.

8. Verfahren zum Herstellen einer Feder wie nach einem der Ansprüche 1 bis 7 definiert, **dadurch gekennzeichnet, dass** es Folgendes umfasst:
• einen Schritt eines Kaltverformens eines Zubehörteils aus einer metastabilen β-Titanlegierung bei einer Rate von größer als 20 %, wobei die Legierung eine Form eines Barrens besitzt und Niob in einem Massenverhältnis von 25 bis 45 %, Zirkonium in einem Massenverhältnis von 0 bis 20 % umfasst, und Sauerstoff in einem Massenverhältnis aufweist, das kleiner als 1,5 % ist, wobei die Struktur anfänglich eine austenitische Phase oder eine martensitische Phase oder eine Mischung der zwei Phasen ist,
• einen Wärmebehandlungsschritt, der eine Aufrechterhaltung des Zubehörteils auf einer Temperatur von 250 bis 550 °C umfasst, während eines Zeitraums, der kleiner als 10 Minuten ist, gefolgt von einer Härtung des Prüfstücks des Zubehörteils, so dass die Legierung nach der Härtung ferner eine ω-Phase von Niederschlägen umfasst.
das Zubehörteil aus der metastabilen β-Titanlegierung, das aus einem kieferorthopädischen Draht (1) aufgebaut ist, und
dadurch, dass das Herstellungsverfahren ferner einen Schritt des Wickelns des kieferorthopädischen Drahtes (1) auf einen Dorn bei Raumtemperatur aufweist und der Schritt des Wickelns dem Wärmebehandlungsschritt vorausgeht, der vorgenommen wird, wenn sich der kieferorthopädische Draht auf dem Dorn befindet.

9. Herstellungsverfahren nach Anspruch 8, **dadurch gekennzeichnet, dass** der Härtungsschritt einen Drahtziehschritt aufweist, dem optional ein Walzschritt vorausgeht.

10. Herstellungsverfahren nach Anspruch 8 oder 9, **dadurch gekennzeichnet, dass** es ferner einen Homogenisierungsschritt des Barrens während 10 Stunden bei 100 °C, gefolgt von einem Härtungsschritt des homogenisierten Barrens in Wasser, aufweist.

11. Herstellungsverfahren nach einem der Ansprüche 8 bis 10, **dadurch gekennzeichnet, dass** der Härtungsschritt einen Schritt einer echten Verbiegung von 4 bis 5 aufweist.

12. Herstellungsverfahren nach einem der Ansprüche 8 bis 11, **dadurch gekennzeichnet, dass** die schraubenförmige kieferorthopädische Feder zwei einander gegenüberliegende Enden aufweist und dass das Herstellungsverfahren ferner einen Schritt eines Schraubens der Befestigungen (2) an jedem Ende der schraubenförmigen kieferorthopädischen Feder aufweist.

## Claims

1. Orthodontic spring made of metastable β titanium alloy, the alloy comprising niobium, zirconium, and oxygen, and optionally molybdenum and tin, **characterized in that** the orthodontic spring is helical, and **in that** the titanium alloy comprises niobium in a mass ratio of between 25 and 45%, zirconium in a mass ratio of between 0 and 20%, and oxygen in a mass ratio of less than 1.5%, the crystallographic structure of the alloy being a martensitic phase with the presence of an austenitic phase and ω-phase precipitates.

2. Orthodontic spring according to claim 1, **characterized in that** it is suitable for operation with variations in tensile forces of less than 10% in a temperature range of from 5°C to 55°C.

3. Orthodontic spring according to either claim 1 or claim 2, **characterized in that** it has two mutually opposite ends and fasteners (2) attached to each end.

4. Orthodontic spring according to claim 3, **characterized in that** each fastener (2) is mounted by screwing on the corresponding end of the orthodontic spring.

5. Orthodontic spring according to claim 3, **characterized in that** each fastener (2) comprises:
• a cavity (21) in which the corresponding end of the orthodontic spring is attached and
• an eye (22) suitable for being to be attached to a hook.

6. Orthodontic spring according to any of claims 1 to 5, **characterized in that** it is formed by an orthodontic wire (1) made of metastable β titanium alloy, the orthodontic wire (1) being wound in a helical shape.

7. Orthodontic spring according to claim 6, **characterized in that** the orthodontic wire (1) has a diameter of 0.18 mm.

8. Method for manufacturing a spring as defined according to any of claims 1 to 7, **characterized in that** it comprises:
• a step of work hardening at a rate greater than 20% of an accessory made of metastable β titanium alloy, the alloy being in the form of an ingot comprising niobium in a mass ratio of between 25 and 45%, zirconium in a mass ratio of between 0 and 20%, and oxygen in a mass ratio of less than 1.5%, the structure initially being an austenitic phase or a martensitic phase or a mixture of the two phases,
• a heat treatment step comprising maintenance of the accessory at a temperature of between 250 and 550°C for a period of less than 10 minutes, followed by quenching of the accessory sample such that the alloy further comprises a ω phase in precipitates after the quenching.
the accessory made of metastable β titanium alloy consisting of an orthodontic wire (1), and
**in that** said manufacturing method further comprises a step of winding the orthodontic wire (1) on a mandrel at room temperature, the winding step preceding the heat treatment step carried out when the orthodontic wire is on the mandrel.

9. Manufacturing method according to claim 8, **characterized in that** the work hardening step comprises a drawing step, optionally preceded by a rolling step.

10. Manufacturing method according to either claim 8 or claim 9, **characterized in that** it further comprises a step of homogenizing the ingot for 10 hours at 100°C, followed by a step of quenching the homogenized ingot in water.

11. Manufacturing method according to any of claims 8 to 10, **characterized in that** the work hardening step comprises a step of true deformation between 4 and 5.

12. Manufacturing method according to any of claims 8 to 11, **characterized in that** the helical orthodontic spring has two mutually opposite ends, and **in that** the manufacturing method further comprises a step of screwing the fasteners (2) to each end of the helical orthodontic spring.
